# EUROPEAN PATENT SPECIFICATION

(11) **EP 0 995 796 B1**
(45) Date of publication and mention of the grant of the patent: **10.05.2006**
(21) Application number: 99119007.5
(22) Date of filing: 04.03.1993
(51) Int. Cl.: C12N 9/02

(54) **Use of Aspergillus niger catalase-R for hydrogen peroxide neutralization**
Verwendung von Aspergillus niger catalase-R zur Wasserstoffperoxid-neutralisierung
Neutralisation du peroxide d'hydrogène par la catalase-R d'Aspergillus niger

(30) Priority: 04.03.1992 US 845990
(43) Date of publication of application: 26.04.2000
(62) Divisional of application: 93907259.1
(73) Proprietor: GENENCOR INTERNATIONAL, INC., South San Francisco, CA 94080 (US)
(72) Inventor: Berka, Randy M., Davis, CA 95616 (US); Fowler, Timothy, So. San Francisco, CA 94080 (US); Vaha-Vahe, Pekka, Hanko (FI)
(74) Representative: Wibbelmann, Jobst

(56) References cited:
- WITTEVEEN C F B ET AL: "LOCALIZATION OF GLUCOSE OXIDASE AND CATALASE ACTIVITIES IN ASPERGILLUS-NIGER" APPLIED AND ENVIRONMENTAL MICROBIOLOGY, vol. 58, no. 4, 1992, pages 1190-1194, XP001056927 ISSN: 0099-2240
- VAN DIJKEN, JOHANNES PIETER ET AL: "Cytochemical localization of glucose oxidase in peroxisomes of Aspergillu niger" EUR. J. APPL. MICROBIOL. BIOTECHNOL. (1980), 9(4), 275-83, XP001056906
- COHEN G ET AL: "SEQUENCE OF THE SACCHAROMYCES-CEREVISIAE CTA1 GENE AND AMINO ACID SEQUENCE OF CATALASE A DERIVED FROM IT" EUROPEAN JOURNAL OF BIOCHEMISTRY, vol. 176, no. 1, 1988, pages 159-164, XP001055464 ISSN: 0014-2956
- CHARY P ET AL: "EVIDENCE FOR THREE DIFFERENTIALLY REGULATED CATALASE GENES IN NEUROSPORA-CRASSA EFFECTS OF OXIDATIVE STRESS HEAT SHOCK AND DEVELOPMENT" JOURNAL OF BACTERIOLOGY, vol. 171, no. 5, 1989, pages 2646-2652, XP001061831 ISSN: 0021-9193
- HEARN V M ET AL: "ANALYSIS OF ASPERGILLUS-FUMIGATUS CATALASES POSSESSING ANTIGENIC ACTIVITY" JOURNAL OF MEDICAL MICROBIOLOGY, vol. 36, no. 1, 1992, pages 61-67, XP001055440 ISSN: 0022-2615
- OKADA H ET AL: "CATALASE GENE OF THE YEAST CANDIDA-TROPICALIS SEQUENCE ANALYSIS AND COMPARISON WITH PEROXISOMAL AND CYTOSOLIC CATALASES FROM OTHER SOURCES" EUROPEAN JOURNAL OF BIOCHEMISTRY, vol. 170, no. 1-2, 1987, pages 105-110, XP001055422 ISSN: 0014-2956
- MOSAVI-MOVAHEDI A.A. ET AL: 'Characterization of Aspergillus niger catalase' INT. J. BIOL. MACROMOL. vol. 9, 1987, pages 327 - 332
- WASSERMANN B.P. ET AL: 'Effect of deglycosylation on the stability of Aspergillus niger catalase' ARCHIVES OF BIOCHEMISTRY AND BIOPHYSICS vol. 212, no. 2, 1981, pages 385 - 392
- GRUFT H. ET AL: 'Properties of a unique catalase isolated from Aspergillus niger' CAN.J.BIOCHEM. vol. 56, 1978, pages 916 - 919
- KIKUCHI-TORII K. ET AL: 'Properties of Aspergillus niger catalase' J. BIOCHEM vol. 92, 1982, pages 1449 - 1456

### SEQUENCE LISTING

<110> GENENCOR INTERNATIONAL, INC.
<120> Use of Aspergillus niger catalase-R for hydrogen peroxide neutralization
<130> EP-83 252
<140> 99119007.5
   <141> 1999-09-28
<150> EP 93907259.1
   <151> 1993-03-04
<160> 6
<170> PatentIn Ver. 2.1
<210> 1
   <211> 3108
   <212> DNA
   <213> Aspergillus niger
<220>
   <221> 5'UTR
   <222> (1) .. (326)
<220>
   <221> exon
   <222> (327)..(620)
<220>
   <221> intron
   <222> (621)..(682)
<220>
   <221> exon
   <222> (683)..(796)
<220>
   <221> exon
   <222> (801)..(908)
<220>
   <221> intron
   <222> (909) .. (969)
<220>
   <221> exon
   <222> (970) .. (1386)
<220>
   <221> intron
   <222> (1387) .. (1440)
<220>
   <221> exon
   <222> (1441)..(1605)
<220>
   <221> intron
   <222> (1606)..(1654)
<220>
   <221> exon
   <222> (1655)..(2743)
<220>
   <221> 3'UTR
   <222> (2744)..(3108)
<220>
   <221> gene
   <222> (1)..(3108)
   <223> Portion of the A. niger catR gene including the coding region
<400> 1
<210> 2
   <211> 730
   <212> PRT
   <213> Aspergillus niger
<400> 2
   Met Arg His Phe Trp Leu Leu Pro Ala Val Ala Gly Ile Ala Gly Ala
    1               5                  10                  15
   Gln Cys Pro Tyr Leu Ser Gly Glu Met Ser Phe Thr Gln Glu Gln Asp
               20                  25                  30
   Asn Ala Gly Asp Thr Ile Glu Val Thr Glu Gln Pro Ile Asp Asn Thr
           35                  40                  45
   Leu Tyr Val Asn Asp Thr Gly Ser Tyr Met Thr Thr Asp Phe Gly Thr
       50                  55                  60
   Pro Ile Ser Asp Gln Thr Ser Leu Lys Ala Gly Pro Arg Gly Pro Thr
   65                  70                  75                  80
   Leu Leu Glu Asp Phe Ile Phe Arg Gln Lys Leu Gln Arg Phe Asp His
                   85                  90                  95
   Glu Arg Val Pro Glu Arg Val Val His Ala Arg Gly Xaa Gly Ala Tyr
               100                 105                 110
   Gly Thr Phe Lys Ser Tyr Ala Asp Trp Ser Asn Val Thr Ala Ala Asp
           115                 120                 125
   Phe Leu Ser Ala Asn Asp Lys Glu Thr Pro Met Phe Cys Arg Phe Ser
       130                 135                 140
   Thr Val Val Gly Phe Arg Gly Ser Val Asp Thr Ala Arg Asp Val His
   145                 150                 155                 160
   Gly His Ala Cys Arg Phe Tyr Thr Asp Glu Gly Asn Tyr Asp Ile Val
                   165                 170                 175
   Gly Ile Asn Phe Ala Pro Phe Phe Ile Gln Asp Ala Ile Gln Phe Pro
               180                 185                 190
   Asp Leu Val His Ala Ile Lys Pro Met Pro Asn Asn Glu Ile Pro Gln
           195                 200                 205
   Ala Ala Thr Ala His Thr Ser Ala Trp Asp Phe Phe Ser Gln Gln Ser
       210                 215                 220
   Thr Ala Leu His Ser Ala Leu Trp Leu Met Ser Gly Asn Gly Ile Pro
   225 230 235 240
   Arg Ser Phe Arg His Met Asn Gly Tyr Gly Val His Ser Phe Arg Phe
   245 250 255
   Val Ala Ala Asn Gly Thr Ser Lys Val Val Arg Thr Pro Trp Lys Ser
   260 265 270
   Gln Gln Gly Val Ala Ser Leu Val Trp Asp Glu Ala Gln Ala Ala Ala
   275 280 285
   Gly Lys Asn Ser Asp Tyr His Arg Gln Asp Leu Tyr Asn Ala Met Pro
   290 295 300
   Asn Gly His Tyr Pro Lys Tyr Glu Leu Gln Ala Gln Ile Met Asp Glu
   305 310 315 320
   Ala Asp Met Leu Arg Phe Gly Phe Asp Leu Leu Asp Pro Thr Lys Leu
   325 330 335
   Val Pro Glu Glu Val Val Pro Tyr Thr Pro Leu Gly Met Met Glu Leu
   340 345 350
   Asn Ala Asn Pro Thr Asn Tyr Phe Ala Glu Val Glu Gln Ala Gly Phe
   355 360 365
   Gln Pro Gly His Val Val Pro Gly Ile Asp Phe Thr Asp Asp Pro Leu
   370 375 380
   Leu Gln Gly Arg Leu Phe Ser Tyr Leu Asp Thr Gln Leu Thr Arg His
   385 390 395 400
   Gly Gly Pro Asn Phe Glu Gln Ile Pro Val Asn Arg Pro Arg Lys Pro
   405 410 415
   Val His Asn Asn Asn Arg Asp Gly Phe Gly Gln Gln Gln Ile Pro Thr
   420 425 430
   Asn Asn Trp Ala Tyr Thr Pro Asn Ser Met Ser Asn Gly Tyr Pro Met
   435 440 445
   Gln Ala Asn Gln Thr Gln Gly His Gly Phe Phe Thr Ala Pro Tyr Arg
   450 455 460
   Tyr Ala Ser Gly His Leu Val Arg Gln Thr Ser Pro Thr Phe Asn Asp
   465 470 475 480
   His Trp Ser Gln Pro Ala Met Phe Trp Asn Ser Leu Ile Pro Ala Glu
   485 490 495
   Gln Gln Met Val Val Asn Ala Ile Val Phe Glu Asn Ser Lys Val Asn
   500 505 510
   Ser Pro His Val Arg Lys Asn Val Val Asn Gln Leu Asn Met Val Asn
   515 520 525
   Asn Asn Leu Ala Val Arg Val Ala Arg Gly Leu Gly Leu Asp Glu Pro
   530 535 540
   Ser Pro Asn Pro Thr Tyr Tyr Thr Ser Asn Lys Thr Ser Asn Val Gly
   545 550 555 560
   Thr Phe Gly Lys Pro Leu Leu Ser Ile Glu Gly Leu Gln Val Gly Phe
                   565                 570                 575
   Leu Ala Ser Asn Ser His Pro Glu Ser Ile Lys Gln Gly Gln Ala Met
               580                 585                 590
   Ala Ala Gln Phe Ser Ala Ala Gly Val Asp Leu Asn Ile Val Thr Glu
           595                 600                 605
   Ala Tyr Ala Asp Gly Val Asn Thr Thr Tyr Ala Leu Ser Asp Ala Ile
       610                 615                 620
   Asp Phe Asp Ala Leu Ile Ile Ala Asp Gly Val Gln Ser Leu Phe Ala
   625                 630                 635                     640
   Ser Pro Ala Leu Ala Asn Gln Met Asn Ser Thr Ala Thr Ser Thr Leu
                   645                 650                 655
   Tyr Pro Pro Ala Arg Pro Phe Gln Ile Leu Val Asp Ser Phe Arg Tyr
               660                 665                 670
   Gly Lys Pro Val Ala Ala Val Gly Ser Gly Ser Val Ala Leu Lys Asn
           675                 680                 685
   Ala Gly Ile Asp Ser Ser Arg Ser Gly Val Tyr Thr Gly Ser Ser Glu
       690                 695                 700
   Thr Thr Glu Lys Ile Ala Lys Glu Val Leu Glu Gly Leu Tyr Thr Phe
   705                 710                 715                     720
   Arg Phe Val Asp Arg Phe Ala Leu Asp Glu
                   725                 730
<210> 3
   <211> 36
   <212> DNA
   <213> Artificial Sequence
<220>
<223> Description of Artificial Sequence: Artificial DNA probe used for cloning the catR gene
<400> 3
   atgttctgga acagcctgat ccccgccgag cagatg                    36
<210> 4
   <211> 36
   <212> DNA
   <213> Artificial Sequence
<220>
   <223> Description of Artificial Sequence:Artificial DNA probe used for cloning the A. niger catR gene
<400> 4
   atgttctgga actccctgat ccccgccgag cagatg 36
<210> 5
   <211> 36
   <212> DNA
   <213> Artificial Sequence
<220>
   <223> Description of Artificial Sequence: Artificial DNA probe used for cloning the A. niger catR gene
<400> 5
   atgttctgga acagcttgat ccccgccgag cagatg 36
<210> 6
   <211> 13
   <212> PRT
   <213> Artificial Sequence
<220>
   <223> Description of Artificial Sequence: Peptide from A. niger catalase-R used for designing a pool of synthetic oligonucleotides for cloning the A. niger catR gene
<400> 6
   Met Phe Trp Asn Ser Leu Ile Pro Ala Glu Gln Gln Met
   1 5 10

## Description

This is a divisional application to the European patent application 93 907 259.1.

### Field of the Invention:

The invention relates to the application of *Asperglllus niger* catalase-A enzyme for the neutralization of hydrogen peroxide in solution.

### Background of the Invention:

Catalases [hydrogen peroxide: hydrogen peroxide oxidoreductases (EC 1.11.1.6)] are enzymes which catalyze the conversion of hydrogen peroxide (H₂O₂) to oxygen (O₂) and water (H₂O) according to the following formula: 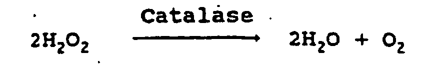 These ubiquitous enzymes have been purified from a variety of animal tissues, plants and microorganisms (Chance and Maehly 1955 Methods Enzymol. **2**: 764-791; Jones and Wilson 1978 in H. Sigel (ed.), *Metal Ions in Biological Systems*, Vol. 7, Marcel Dekker Inc., New York). Nearly all forms of the enzyme which have been characterized consist of four polypeptide subunits, each having a molecular weight of 50,000 to 60,000 and containing one protohemin prosthetic group per subunit (Wasserman and Hultin 1981 Arch. Biochem. Biophys. **212**: 385-392; Hartig and Ruis 1986 Eur. J. Biochem. **160**: 487-490).

Bovine liver catalase has been the most extensively studied variety of this enzyme [Schonbaum and Chance 1976 in *The Enzymes* (P.D. Boyer, ed.) 3rd edn., vol. 13, pp. 363-408, Academic Press, New York]. The complete amino acid sequence and three dimensional structure of bovine liver catalase are known (Schroeder, et al., 1982 Arch. Biochem. Biophys. **214**: 397-412; Murthy, et al., 1981 J. Mol. Biol. **152**: 465-499).

Although less well-studied from a biochemical and biophysical standpoint, catalases from filamentous fungi have several characteristics that distinguish them from their mammalian counterparts. While similar in subunit number and heme content, fungal catalases are substantially larger molecules than those from other organisms, having subunit molecular weights ranging from 80,000 to 97,000 (Vainshtein, et al., 1986 J. Mol. Biol. **188**: 63-72; Jacob and Orme-Johnson 1979 Biochem. **18**: 2967-2975; Jones, et al., 1987 Biochim. Biophys. Acta **913**: 395-398; Mosavi-Movahedi, et al., 1987 Int. J. Biol. Macromol **9:** 327-332; Wasserman and Hultin, 1981 Arch. Biochem. Biophys. **212:** 385-392; Gruft, et al., 1978 Can. J. Biochem. **56:** 916-919; Kikuchi-Torii, et al., 1982 J. Biochem. **92:** 1449-1456). More importantly, catalases from fungi such as *Aspergillus niger* are more stable than beef liver catalase to proteolysis and to inactivation by glutaraldehyde, SDS, and have lower affinity for catalase inhibitors such as cyanide, azide and fluoride (Wasserman and Hultin 1981 Arch. Biochem. Biophys**. 212**: 385-392). In addition, *A. niger* catalase is significantly more stable than bovine liver catalase when subjected to extremes of pH, hydrogen peroxide, and temperature (Scott and Hammer 1960 Enzymologia **22**: 229-237). Although fungal catalases offer stability advantages, the corresponding mammalian enzymes such as beef liver catalase generally have higher catalytic activity (Gruft, et al., 1978; Can. J. Biochem. **56:** 916-919). Traditionally, beef liver catalase has been the preferred enzyme for diagnostic purposes and for pharmaceutical-related applications (e.g., contact-lens cleaning/disinfection/H₂O₂ neutralization). However, recent outbreaks of a slow-virus disease known as BSE (bovine spongiform encephalopathy) in European cattle herds and fear that this disease might be spread to man [Dealler and Lacey 1991 Nutr. Health (Bicester) **7**: 117-134; Dealler and Lacey 1990 Food Microbiol. **7**: 253-280] have aroused interests in finding alternatives to beef liver catalase for these applications. Furthermore, since enzyme stability is an important factor in the biotechnological utilization of enzymes, there is considerable interest in the use of *A. niger* catalase, in applications involving neutralization of high concentrations of hydrogen peroxide and for pharmaceutical-related applications in which the use of bovine liver catalase could be a health risk.

Although there are a number of published reports describing the biochemical and biophysical properties of *A. niger* catalase, it has not been previously disclosed that there are in fact two catalase enzymes present in this organism. Using standard molecular biology techniques, we have isolated genes (*catA* and *catR*) encoding two different catalase enzymes (catalase-A and catalase-R) from *A. niger* (Genencor International, Inc., unpublished). The *A. niger catA* gene, cloned by cross-hybridization to the yeast (*Saccharomyces cerevisiae*)

*CTA1* gene, encodes a catalase enzyme which is induced primarily during growth on fatty acids and is presumably peroxisomal. In contrast to the reported subunit molecular weights of catalases ranging from 80,000 to 97,000 it was found that the subunit molecular weight of catalase-A from *Aspergillus niger* is approximately 46,000 as determined by SDS-PAGE. *A. niger* catalase-A is less stable (i.e., has a shorter half-life in storage), is inactivated more rapidly by high concentrations of hydrogen peroxide, and less active than catalase-R at low pH. The *A. niger catR* gene encodes a soluble cytoplasmic enzyme (catalase-R) which we discovered to be the major activity in commercial catalase preparations.

### Summary of the Invention:

It has been discovered that the *A. niger* genome contains two catalase genes, designated *catA* and *catR*. In addition, it was discovered that the *catA* and *catR* gene products (catalase-A and catalase-R, respectively) are two different catalase enzymes, each having their own distinctive characteristics.

The present invention relates to a catalase-A enzyme for the neutralization of hydrogen peroxide in solution characterized by a subunit weight of approximately 46,000 as determined by SDS-PAGE, a specific activity of 6300 IU/mg and in that the synthesis thereof is induced by the presence of fatty acids.

### Figures:

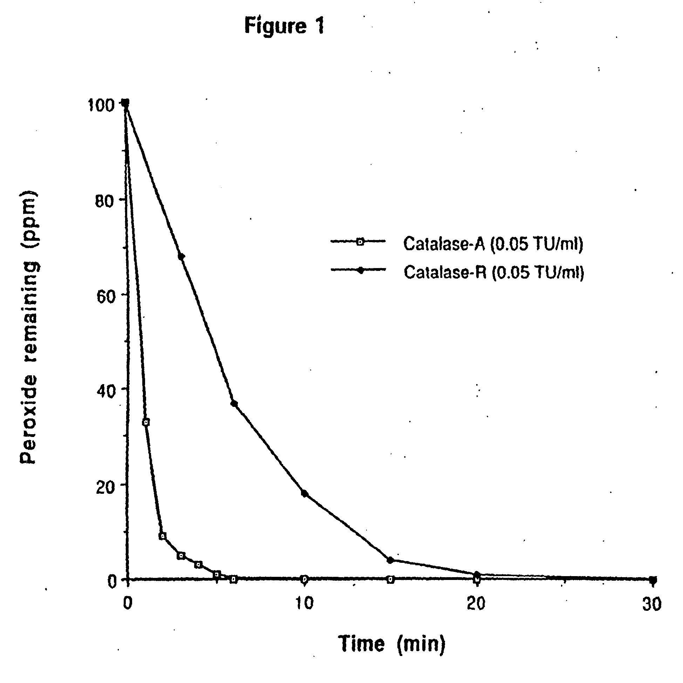

Figure 1 displays a comparison of catalase-A and catalase-R activities in dilute hydrogen peroxide solution (100 ppm).
Figure 2 shows a comparison of catalase-A and catalase-R activities in concentrated hydrogen peroxide solution (16.5 g/L).

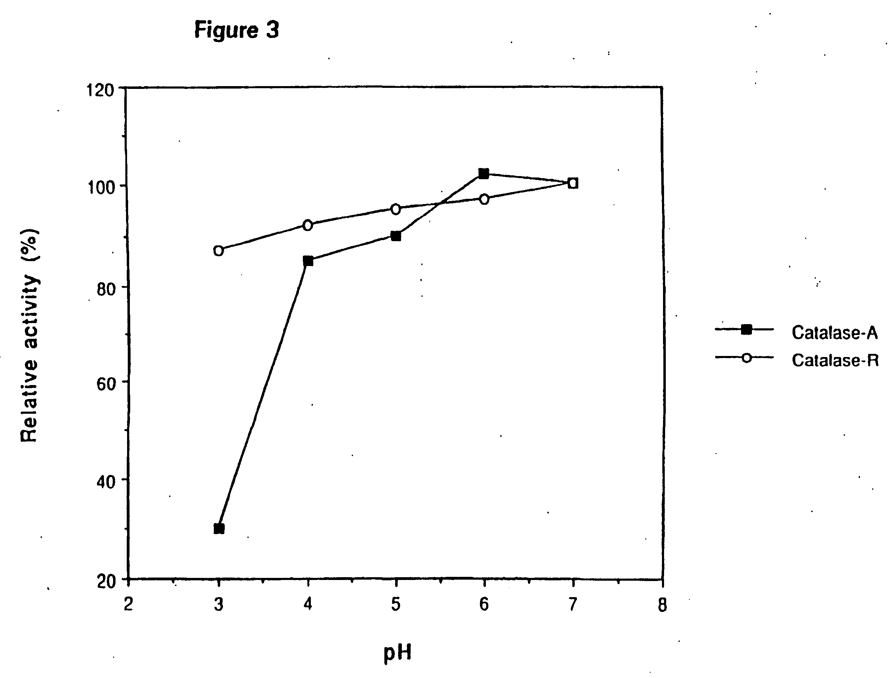

Figure 3 illustrates a comparison of enzymatic activities of catalase-A and catalase-R at different pH values.
Figure 4 shows restriction maps of the *A. niger catA* and *catR* genes encoding catalase-A and catalase-R, respectively.
Figures 5 A-C display the nucleotide sequence and deduced amino acid sequence of the *A. niger catR* gene. Introns are denoted by dashed lines.

### Description of the Preferred Embodiments.

A comparison of the biochemical and biophysical properties of catalase-A and catalase-R is described below. One skilled in the art will understand that various changes in the following examples could be made. Accordingly, the examples are not intended to be limiting.

### 1. Comparison of the biochemical and biophysical properties of catalase-A and catalase-R.

Techniques used to measure biochemical parameters such as enzyme activity, hydrogen peroxide concentrations, pH-activity profile, and enzyme stability are conventional techniques described in the following examples.

### Purification of A. niger catalase-R

Catalase-R was purified from a commercial preparation of *A*. *niger* catalase (Fermcolase 1000, Genencor International, Inc.) using ion exchange chromatography on TrisAcryl Q resin (IBF Biotechnics, Inc.). First, the commercial enzyme preparation was desalted on a PD-10 column (Pharmacia-LKB) equilibrated in 10 mM Tris-HC1 at pH 8. The enzyme was loaded onto a 2.5 cm x 5.5 cm TrisAcryl Q column equilibrated first with 100 ml of 100 mM Tris-HC1, pH 8, then with 200 ml of 10 mM Tris-HC1, pH 8 at a flow rate of 6.0 ml/min. Unbound proteins were eluted by washing the column with 10 mM Tris-HC1. The remaining proteins were eluted with a gradient of increasing NaCl (zero to 0.5 M in 10 mM Tris-HC1, pH 8). The protein fraction having the highest A₄₁₀/A₂₈₀ ratio contained purified catalase. SDS-PAGE analysis of this protein fraction showed a single-band which migrated with an apparent molecular weight of approximately **80,000.**

### Purification of A. niger catalase-A

*A. niger* catalase-A was purified from mycelial extracts of cells grown for two days in medium which contained 1% glucose as the carbon source. The mycelia were quick-frozen in liquid nitrogen and ground to a fine powder in an electric coffee-grinder. The frozen powder was suspended in 10 ml of 100 mM sodium formate buffer, pH 7 and kept on ice for one hour. The insoluble debris was removed by centrifugation, and the supernatant was concentrated by ultrafiltration (Amicon). The concentrated extract was then chromatographed on Sephacryl S-100 and the fractions with catalase activity were rechromatographed on an ion exchange column as described above. Two peaks, one major and one minor, containing catalase activity were analyzed by SDS-PAGE. The major catalase fraction gave a predominant band with a molecular weight of 80,000 and was subsequently shown to be catalase-R. The minor component, catalase-A, gave a band with molecular weight of 46,000.

Table 1 shows a comparison of the biochemical and biophysical properties of *A. niger* catalase-A and catalase-R.

**Table 1**

| Properties | Catalase-R | Catalase-A |
|---|---|---|
| Molecular weight | 80,000 | 46,000 |
| Specific activity (IU/mg) | 7500 | 6300 |
| Synthesis induced by | glucose | fatty acids |
| Cellular location | cytoplasmic | peroxisomal |
| Stability to high H₂O₂ | yes | no |
| Storage stability (35°C, 3 days, pH 7) | 55% | >90% |

### 2. Cloning of the A. niger catA and catR genes.

The techniques used in cloning the *A. niger catA* and *catR* genes are conventional techniques described in Sambrook, et al., 1989 *Molecular Cloning, A Laboratory Manual*, Cold Spring Harbor Press, Cold Spring Harbor, NY.

### Cloning of the A. niger catR gene

A series of proteolytic fragments were generated from purified catalase-R by digestion of the protein with cyanogen bromide. These peptide fragments were subjected to amino acid sequence analysis. The amino acid sequence information was employed to design synthetic DNA probes for identification of *catR*-specific cDNA sequences in a λgt11 library. Briefly, the peptide fragment Met-Phe-Trp-Asn-Ser-Leu-Ile-Pro-Ala-Glu-Gln-Gln-Met was used to design a pool of three synthetic oligonucleotides having the following sequences:

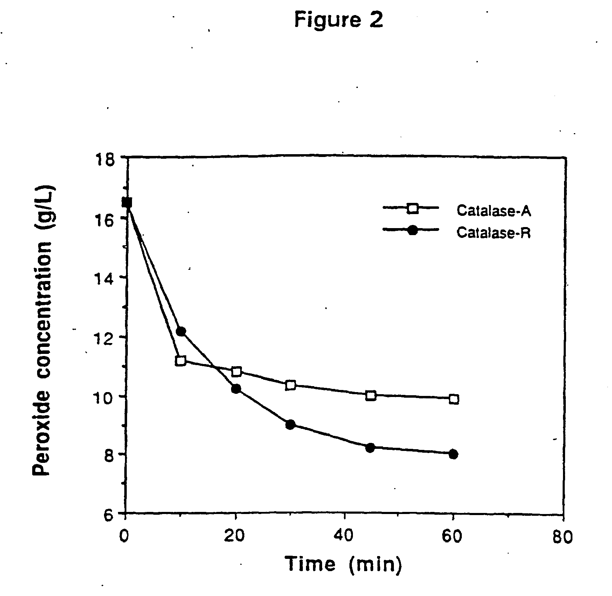

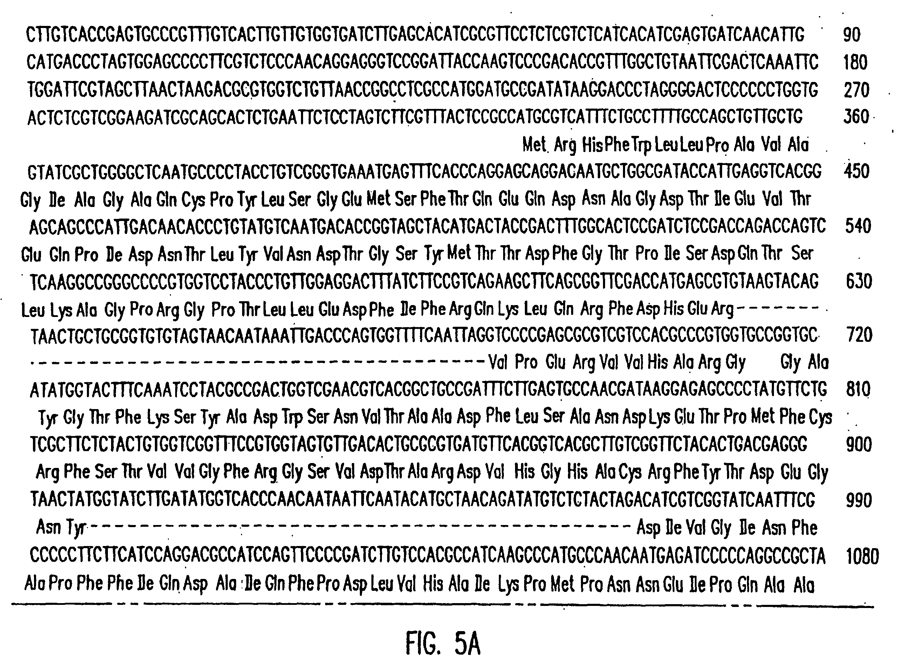

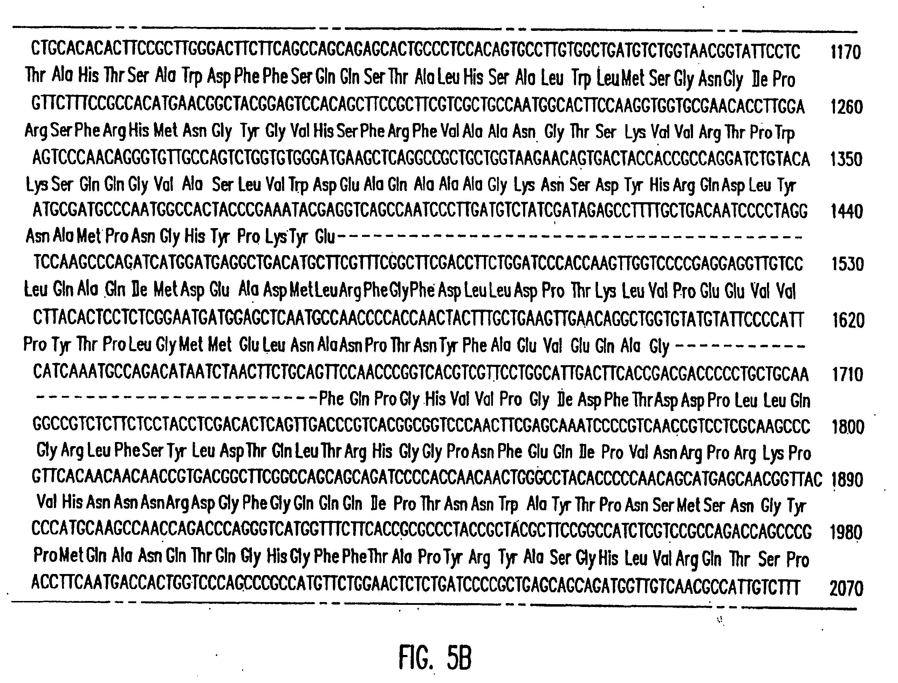

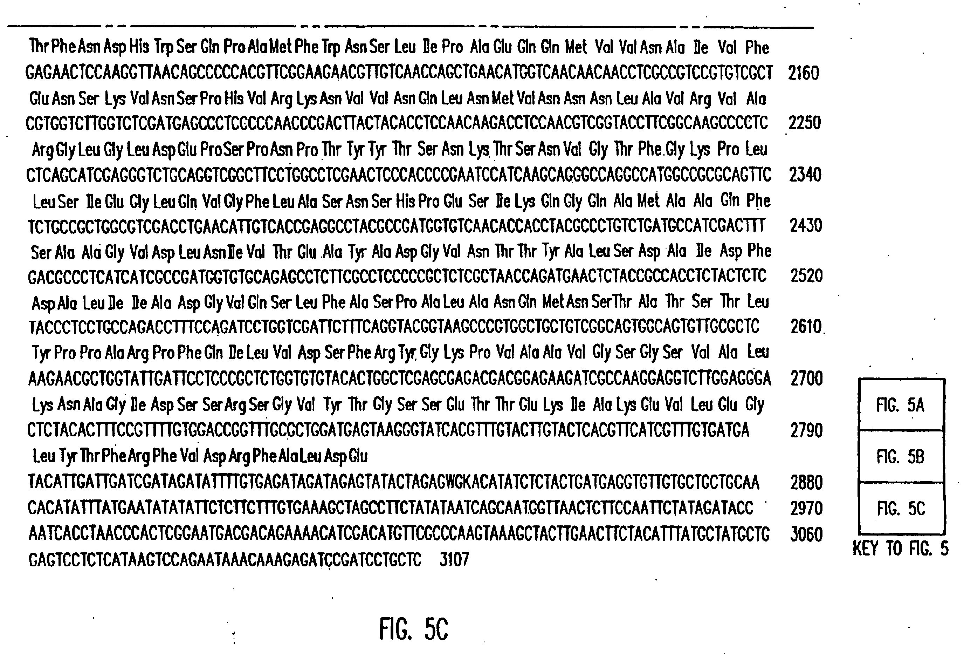

This peptide was chosen because the amino acids give minimally degenerate codon choices. The differences among the three synthetic oligonucleotides represent alternate codon choices where there was no strong bias in the known codon usage pattern for *A. niger*. This position of this proteolytic fragment corresponds to peptide 3 shown in Figure 2. A clone containing a partial cDNA fragment was positively identified by hybridization with the synthetic DNA probe and nucleotide sequence analysis of this clone confirmed that it encoded catalase-R. This cloned cDNA segment was used to probe a library of *A. niger* genomic DNA. Subsequently, the entire *catR* gene, plus upstream and downstream transcriptional control elements, was assembled as a 9.0 kb *Hind*III - *Xho*I restriction fragment. The nucleotide sequence of the *catR* coding region has been determined and is given in Figure 5.

### Cloning of the A. niger catA gene

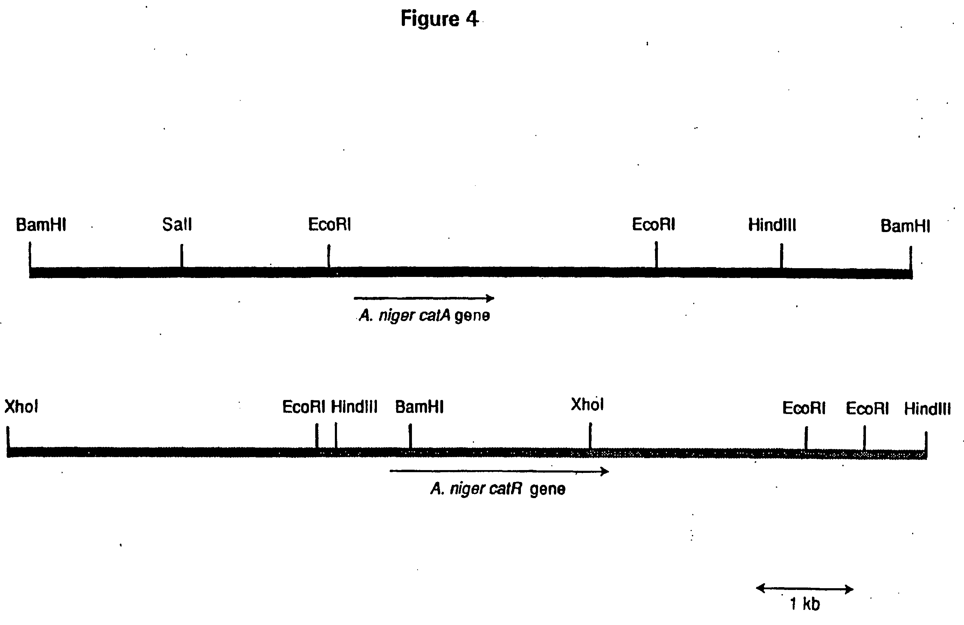

The *A. niger catA* gene was cloned by cross-hybridization using the *S*. *cerevisiae CTA1* gene as the probe. The yeast catalase genes have been previously isolated and their sequences published (Cohen, et al., 1988 Eur. J. Biochem. **176**: 159-163; Hartig and Ruiz 1986, Eur. J. Biochem. **160**: 487-490). A fragment of the yeast *CTA1* gene was amplified using PCR (Perkin Elmer-Cetus) and cloned into pUC18. Sequence analysis of this clone confirmed it to be the yeast peroxisomal catalase gene. The clone was then used to screen Southern blots of *A. niger* FS-1 genomic DNA and found to hybridize to a single *Eco*RI band of 3.2 kb. The probe was then used to screen a λEMBL library of FS-1 genomic DNA, resulting in the identification of several positive clones, each containing the previously identified 3.2 kb *Eco*RI restriction fragment. One of these λEMBL clones (λEMBL3-1) was digested with *Bam*HI and a 9 kb fragment was subcloned into pUC18. A restriction map of this subclone is given in Figure 4. DNA sequence analysis of a portion of this fragment which hybridized with the yeast *CTA1* gene showed that the clone encoded a catalase that was different than catalase-R. On the basis of its homology with the yeast *CTA1* gene, it was given the designation *catA*. Co-transformations of *A. niger* FS-1 were done using the pUC18-*catA* plasmid and a plasmid containing a benomyl-resistance conferring β-tubulin gene. Benomyl-resistant colonies were screened for increased number of integrated *catA* gene copies by Southern blotting. Those transformants which showed more than one *catA* gene copy were evaluated in shake flask cultures. One transformant, - designated 208, showed an approximate doubling in total cellular catalase activity.

## Claims

1. A catalase A enzyme derivable from *Aspergillus niger* being **characterized by** a subunit weight of approximately 46,000 as determined by SDS-PAGE, a specific activity of 6300 IU/mg and in that the synthesis thereof is induced by the presence of fatty acids.

## Patentansprüche

1. Katalase A-Enzym, isolierbar aus *Aspergillus niger*, das durch ein Untereinheiten-Gewicht von etwa 46.000, wie durch SDS-PAGE bestimmt, eine spezifische Aktivität von 6300 IE/mg und **dadurch** charakterisiert ist, dass seine Synthese durch die Anwesenheit von Fettsäuren induziert wird.

## Revendications

1. Enzyme catalase A pouvant être obtenue à partir de *Aspergillus niger* **caractérisée par** un poids moléculaire de la sous-unité, déterminé par SDS-PAGE, d'environ 46 000 Da, une activité spécifique de 6300 UI/mg et en ce que sa synthèse est induite par la présence d'acides gras.
